# EUROPEAN PATENT APPLICATION

(11) **EP 1 842 436 A1**
(43) Date of publication of application: **10.10.2007**
(21) Application number: 06711933.9
(22) Date of filing: 19.01.2006
(51) Int. Cl.: A23L 1/236, C07C 31/26

(54) **SUGAR ALCOHOL EUTECTIC AND PROCESS FOR PRODUCING THE SAME**

(30) Priority: 24.01.2005 JP 2005014975; 25.04.2005 JP 2005125948
(71) Applicant: Nikken Fine Chemical Co., Ltd., Chita-shi Aichi 4780046 (JP)
(72) Inventor: OKAZAKI, Satoshi, ma-cho, Chita-shi Aichi, 4780046 (JP); TODA, Yoshinori, ma-cho, Chita-shi Aichi, 4780046 (JP); NAKAMURA, Yoshinobu, ma-cho, Chita-shi Aichi, 4780046 (JP); SHINJI, Kazunori, ma-cho, Chita-shi Aichi, 4780046 (JP); URAJI, Tatsuya, ma-cho, Chita-shi Aichi, 4780046 (JP)
(74) Representative: Gille Hrabal Struck Neidlein Prop Roos
(86) International application number: PCT/JP2006/300687
(87) International publication number: WO 2006/077904

(57) **Abstract**

To provide an eutectic crystalline sugar alcohol representing a single melting peak obtained by differential scanning calorimetry, which does not show any one of problems of requiring the addition of a comparatively large amount of a seed crystal to be added for crystallization and a prolonged aging period of several days for crystal growth as well as problems in production, such as an inefficient and complicated production process, while having excellent taste, low hygroscopicity, and excellent uniformity, grindability, and non-consolidation property; and a manufacturing method thereof, which comprises allowing a liquid composition containing two or more kinds of the sugar alcohol at a predetermined ratio to be added with a powder containing crystals of the same two or more kinds of the sugar alcohol at substantially the same usage ratio as that of the sugar alcohol, and kneading and aging a resulting mixture.

## Description

### Technical field

The present invention relates to an eutectic crystalline sugar alcohol and a manufacturing method thereof.

### Background art

Sugar alcohols have excellent properties of non-dental caries, low-calories, and heat resistance, and have been utilized in many food products.
In addition, the sugar alcohols have their respective characteristics with respect to qualities of tastes, which are different from the quality of sweetness of sugar being most widely used. For example, monosaccharide sugar alcohols such as erythritol, xylitol, and sorbitol have characteristics of providing refreshing sweetness and feeling of coolness, but provide sometime feeling of astringent taste. Therefore, it is known that favorable quality of sweetness, which is fairly close to sugar can be attained when the monosaccharide sugar alcohol is used together with disaccharide sugar alcohol or the like.
However, when crystalline powders of two or more kinds of the sugar alcohol are mixed together, segregation may cause owing to differences in sizes and shapes of particles, particle size and specific gravities of the powders. Besides, the sugar alcohols have their respective rates of dissolution, so uneven sweetness may be felt and desirable qualities of tastes may hardly be obtained.
In other words, when two or more kinds of the crystalline powder sugar alcohols are mixed, there occurred problems of segregation and uncoordinated qualities of sweetness.

As a sugar alcohol composition excellent in ability of processing such as uniformity etc., a molten eutectic sorbitol/xylitol, an eutectic mannitol/sorbitol polymorph, a hydrogenated starch hydrolysate powder obtained by kneading after addition of sorbitol and maltitol crystals to a hydrogenated starch hydrolysate mainly composing of sorbitol and maltitol, and a crystalline powder erythritol composition obtained by kneading after addition of a seed crystal to an aqueous solution containing erythritol and a food product or food additive have been reported in some publications (see, for example, Patent Documents 1, 2, 3 and 4).

However, the aforementioned sugar alcohol composition requires a comparatively large amount of the seed crystal for crystallization, an aging period as long as several days for crystal growth, and the like, while having problems of an inefficient and complicated production process and the like. In addition, the sugar alcohol composition thus obtained had problems of poor grindability, high hygroscopicity, and facilitated consolidation.

Furthermore, the hydrogenated starch hydrolysate powder has a disadvantage of being easily consolidated compared with other powdery saccharides, although it provides a refreshing sweetness. Since there occurs a problem of poor workability in use when the powdery product has consolidated, there is a need of preventing the consolidation when the powdery product is distributed. In general, a powdery carbohydrate product is distributed in a craft bag or corrugated cardboard packaging, and also a comparatively consolidable product is usually distributed in a corrugated cardboard packaging.
With respect to the consolidation, in the case of granulated products being classified so as to be of all less than 2 mm in particle size, one which will be consolidated within less than one month in corrugated cardboard packaging is not preferable for a product, but preferable is one that does not consolidate within at least one month, particularly preferable is one that does not consolidate for three months or more.
On the other hand, in the case of powdery products being classified so as to be of all less than 350 µm in particle size, one which will be consolidated within less than two weeks in corrugated cardboard packaging is not preferable for a product, but preferable is one that does not consolidate within at least two weeks, particularly preferable is one that does not consolidate for one month or more.

Patent Document 1: JP 05-201899 A
Patent Document 2: JP 03-72438 A
Patent Document 3: JP 2002-253167 A
Patent Document 4: JP 08-266244 A

### Disclosure of the invention

### Problem to be solved by the invention

An object of the present invention is to provide a powder containing an eutectic crystalline sugar alcohol or an eutectic crystalline sugar alcohol, which does not show any one of problems of requiring the addition of a comparatively large amount of a seed crystal to be added for crystallization and a prolonged aging period of several days for crystal growth as well as problems in production, such as an inefficient and complicated production process, while being low hygroscopic and excellent in uniformity, grindability, and non-consolidation property as well as good feeling of dissolving in the mouth and quality of sweetness.

### Means for solving the problem

As a result of extensive studies made by the inventors of the present invention for solving the problems of the conventional proposals as described above, the present invention has been finally completed by finding out that an eutectic crystalline sugar alcohol can be efficiently produced by defining the blending ratio of a sugar alcohol-containing composition within a specific ratio, while the eutectic crystalline sugar alcohol to be obtained has excellent taste, low hygroscopicity, and excellent uniformity, grindability, and non-consolidation property.
In other words, the inventors of the present invention have found out that an eutectic crystalline sugar alcohol, which represents a single melting peak obtained by differential scanning calorimetry, can be efficiently produced by adjusting the blending ratio of a sugar alcohol-containing composition within in a specific range and the eutectic crystalline sugar alcohol thus obtained has good feeling of dissolving in the mouth and an excellent quality of sweetness and is uniform and does not segregate, while having physical properties of low hygroscopicity and excellent grindability. Therefore, based on such findings, the present invention has been completed.

Furthermore, as a result of extensive study for solving the disadvantages of the hydrogenated starch hydrolysate powder, the consolidation of the product is substantially influenced by three factors of moisture content, a fine powder, and a melting calorie. The present inventors have found that improvement of those factors may improve the consolidation property and completed the present invention based on such findings such findings.
In other words, the inventors of the present invention have found that a powder having an improved consolidation property can be obtained by adjusting the moisture content in the powder containing an eutectic crystalline sorbitol/maltitol (the percentage content of moisture) to 2% by weight or less and/or adjusting the percentage content of fine powder part, more specifically that of fine powders having particle size of less than 150 µm to 30% or less, and/or adjusting the melting calorie thereof to 70 J/g or more. Therefore, based on such findings, the present invention has been completed.

According to a first aspect of the present invention, there is provided a method of manufacturing an eutectic crystalline sugar alcohol that represents a single melting peak obtained by differential scanning calorimetry, comprising: allowing a liquid composition containing two or more kinds of the sugar alcohol at a predetermined ratio to be added with a powder containing crystals of the same two or more kinds of the sugar alcohol at substantially the same usage ratio as that of the sugar alcohol; and kneading and aging a resulting mixture.
According to a second aspect of the present invention, there is provided a method of manufacturing an eutectic crystalline sugar alcohol according to the first aspect, in which the sugar alcohol includes at least one or more kinds of disaccharide sugar alcohol.
According to a third aspect of the present invention, there is provided a method of manufacturing an eutectic crystalline sorbitol/maltitol that represents a single melting peak at a temperature of 91 ± 5°C obtained by differential scanning calorimetry, comprising: allowing a liquid composition containing sorbitol in an amount of 51 to 80% by weight and maltitol in an amount of 49 to 20% by weight to be added with a powder containing a sorbitol crystal and a maltitol crystal at the same usage ratio as that of the sorbitol and the maltitol, respectively; and kneading and aging a resulting mixture.
According to a fourth aspect of the present invention, there is provided an eutectic crystalline sugar alcohol that represents a single melting peak obtained by differential scanning calorimetry, obtained by: allowing a liquid composition containing two or more kinds of the sugar alcohol at a predetermined ratio to be added with a powder containing crystals of the same two or more kinds of the sugar alcohol at substantially the same usage ratio as that of the sugar alcohol; and kneading and aging a resulting mixture.
According to a fifth aspect of the present invention, there is provided an eutectic crystalline sugar alcohol according to the fourth aspect, in which the sugar alcohol includes at least one or more kinds of disaccharide sugar alcohol.
According to a sixth aspect of the present invention, there is provided an eutectic crystalline sorbitol/maltitol that represents a single melting peak at a temperature of 91 ± 5°C obtained by differential scanning calorimetry, obtained by: allowing a liquid composition containing sorbitol in an amount of 51 to 80% by weight and maltitol in an amount of 49 to 20% by weight to be added with a powder containing a sorbitol crystal and a maltitol crystal at the same usage ratio as that of the sorbitol and the maltitol, respectively; and kneading and aging a resulting mixture.
According to a seventh aspect of the present invention, there is provided an eutectic crystalline sugar alcohol that represents a single melting peak obtained by differential scanning calorimetry according to any one of the fourth to sixth aspects, in which the percentage content of the powder having a particle size of less than 350 µm is 95% or more.
According to an eighth aspect of the present invention, there is provided a powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, in which a moisture content is 2% by weight or less.
According to a ninth aspect of the present invention, there is provided a powder containing an eutectic crystalline sorbitol/maltitol , comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, in which the percentage content of a fine powder having a particle size of less than 150 µm is 30% or less.
According to a tenth aspect of the present invention, there is provided a powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, in which a melting calorie is 70J/g or more.
According to an eleventh aspect of the present invention, there is provided a powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, in which a moisture content is 2% by weight or less, and/or a percentage content of a fine powder having a particle size of less than 150 µm is 30% or less, and/or a melting calorie is 70J/g or more.
According to a twelfth aspect of the present invention, there is provided a powder containing an eutectic crystalline sorbitol/maltitol according to any one of the eighth to eleventh aspects, in which the percentage content of the powder having a particle size of less than 350 µm is 95% or more.
According to a thirteenth aspect of the present invention, there is provided a powder containing an eutectic crystalline sorbitol/maltitol according to any one of the eighth to twelfth aspects, in which the content of the sorbitol is 40 to 60% by weight and the content of the maltitol is 60 to 40% by weight, and the content of high molecular sugar alcohol, which is not smaller than maltotriitol, is less than 10% by weight.
According to a fourteenth aspect of the present invention, there is provided a method of manufacturing a composition containing an eutectic crystalline sugar alcohol, comprising an eutectic crystalline sugar alcohol that represents a single melting peak obtained by differential scanning calorimetry, and ingredients of food products, food additives, pharmaceutical excipients, pharmaceutical products, or cosmetic products, in which: a liquid composition containing two or more kinds of the sugar alcohol at a predetermined ratio is added with ingredients of food products, food additives, pharmaceutical excipients, pharmaceutical products, or cosmetic products, followed by addition of a powder containing crystals of the same two or more kinds of the sugar alcohol at substantially the same usage ratio as that of the sugar alcohol, and kneading and aging a resulting mixture; or a liquid composition containing two or more kinds of the sugar alcohol at a predetermined ratio is added with a powder containing crystals of the same two or more kinds of the sugar alcohol at substantially the same usage ratio as that of the sugar alcohol, simultaneously with addition of ingredients of food products, food additives, pharmaceutical excipients, pharmaceutical products, or cosmetic products, followed by kneading and aging.
According to a fifteenth aspect of the present invention, there is provided a method of manufacturing a powdery composition, comprising a powder containing an eutectic crystalline sorbitol/maltitol, and ingredients of food products, food additives, pharmaceutical excipients, pharmaceutical products, or cosmetic products, in which: a liquid composition in which the content of the sorbitol is 40 to 80% by weight and the content of the maltitol is 60 to 20% by weight is added with ingredients of food products, food additives, pharmaceutical excipients, pharmaceutical products, or cosmetic products, followed by addition of a powder containing a sorbitol crystal and a maltitol crystal at the same usage ratio as that of the sorbitol and the maltitol, respectively, and kneading and aging a resulting mixture; or a liquid composition in which the content of the sorbitol is 40 to 80% by weight and the content of the maltitol is 60 to 20% by weight is added with a powder containing a sorbitol crystal and a maltitol crystal substantially at the same usage ratio as that of the sorbitol and maltitol, respectively, simultaneously with addition of ingredients of food products, food additives, pharmaceutical excipients, pharmaceutical products, or cosmetic products, followed by kneading and aging.
According to a sixteenth aspect of the present invention, there is provided a composition containing an eutectic crystalline sugar alcohol which is produced by a method according to the fourteenth aspect, comprising an eutectic crystalline sugar alcohol that represents a single melting peak obtained by differential scanning calorimetry, and ingredients of food products, food additives, pharmaceutical excipients, pharmaceutical products, or cosmetic products.
According to a seventeenth aspect of the present invention, there is provided a powdery composition which is produced by a method according to the fifteenth aspect of the invention, comprising a powder containing an eutectic crystalline sorbitol/maltitol, and ingredients of food products, food additives, pharmaceutical excipients, pharmaceutical products, or cosmetic products.

### Effect of the invention

According to the present invention, there is no need of the addition of a comparatively large amount of a seed crystal to be added for crystallization or a prolonged aging of several days for crystal growth without causing any inefficient and complicated production process or the like, thereby allowing an efficient production of an eutectic crystalline sugar alcohol having a single melting peak obtained by differential scanning calorimetry.
The eutectic crystalline sugar alcohol of the present invention thus obtained is extremely excellent in feeling of dissolving in the mouth, and, by simultaneously dissolving the contained sugar alcohol in the mouth, a feeling of astringent taste of the sugar alcohol can be negated, thereby allowing the quality of sweetness to become preferable with definite feeling.
Furthermore, the eutectic crystalline sugar alcohol of the present invention is homogeneous without any segregation, while having low hygroscopicity as well as having excellent grindability and non-consolidation property.

### Brief description of the drawings

In the accompanying drawings:
[Fig. 1] is a diagram illustrating a DSC curve obtained by a calorimetric analysis on the eutectic crystal obtained in Example 1 using differential scanning calorimetry (DSC);
[Fig. 2] is a diagram illustrating a DSC curve obtained by a calorimetric analysis on the eutectic crystal obtained in Comparative Example 1 using differential scanning calorimetry (DSC);
[Fig. 3] is a diagram illustrating a DSC curve obtained by a calorimetric analysis on the eutectic crystal obtained in Example 2 using differential scanning calorimetry (DSC);
[Fig. 4] is a diagram illustrating a DSC curve obtained by a calorimetric analysis on the eutectic crystal obtained in Example 3 using differential scanning calorimetry (DSC);
[Fig. 5] is a diagram illustrating a DSC curve obtained by a calorimetric analysis on the eutectic crystal obtained in Example 5 using differential scanning calorimetry (DSC);
[Fig. 6] is a diagram illustrating a DSC curve obtained by a calorimetric analysis on the eutectic crystal obtained in Example 6 using differential scanning calorimetry (DSC);
[Fig. 7] is a diagram illustrating a DSC curve obtained by a calorimetric analysis on the eutectic crystal obtained in Example 7 using differential scanning calorimetry (DSC);
[Fig. 8] is a diagram illustrating a DSC curve obtained by a calorimetric analysis on the eutectic crystal obtained in Example 8 using differential scanning calorimetry (DSC);
[Fig. 9] is a diagram illustrating a DSC curve obtained by a calorimetric analysis on the eutectic crystal obtained in Example 9 using differential scanning calorimetry (DSC); and
[Fig. 10] is a diagram illustrating a DSC curve obtained by a calorimetric analysis on the powder containing an eutectic crystalline sorbitol/maltitol obtained in Example 14 with a seed addition rate of 35% by weight using differential scanning calorimetry (DSC).
Best mode for carrying out the invention

The present invention according to the first aspect relates to a method of manufacturing an eutectic crystalline sugar alcohol that represents a single melting peak obtained by differential scanning calorimetry, comprising: allowing a liquid composition containing two or more kinds of the sugar alcohol at a predetermined ratio to be added with a powder containing crystals of the same two or more kinds of the sugar alcohol at substantially the same usage ratio as that of the sugar alcohol; and kneading and aging a resulting mixture.
The term "eutectic crystalline sugar alcohol" as used herein refers to an eutectic crystal having at least two or more kinds of the sugar alcohol being co-crystallized. Examples of such an eutectic crystalline sugar alcohol include an eutectic crystal having two or more kinds of the sugar alcohol, in which two or more kinds thereof are co-crystallized, more specifically including an eutectic crystal having two kinds of the sugar alcohol being co-crystallized and an eutectic crystal having three kinds of the sugar alcohol being co-crystallized.

Furthermore, the term "eutectic crystalline sugar alcohol" as used herein also refers to a mixture of crystals of two or more kinds of the sugar alcohol, which can be simultaneously precipitated from an aqueous phase of the sugar alcohol. The term "eutectic crystal" as used herein is defined on the basis of the description of "eutectic crystal is a mixture of two or more crystals simultaneously precipitated from a liquid phase" in the Comprehensive Chemistry Dictionary, compact edition, 32nd issue, published by KYORITSU SHUPPAN CO., LTD. (August 15, 1989).

Examples of the sugar alcohol include sorbitol, maltitol, erythritol, xylitol, mannitol, inositol, lactitol, α-glucopyranosyl-1,6-mannitol (α-glucopyranosyl-1,1-mannitol), α-glucopyranosyl-1,6-sorbitol, and cellobitol, and maltooligosaccharide alcohols such as maltotriitol and maltotetraitol.

In the present invention according to the first aspect, at least two or more kinds of the sugar alcohols are used. As described in the second aspect, the one containing as a sugar alcohol at least one or more kinds of disaccharide sugar alcohols is preferably used.
Examples of the disaccharide sugar alcohol include maltitol, lactitol, α-glucopyranosyl-1,6-mannitol (α-glucopyranosyl-1,1-mannitol), α-glucopyranosyl-1,6-sorbitol, andcellobitol. Containing at least one or more kinds of disaccharide sugar alcohols makes it possible to provide goodsweetness. Maltitol, α-glucopyranosyl-1,6-mannitol, or α-glucopyranosyl-1,6-sorbitol is particularly preferably used as the disaccharide sugar alcohol.

In the present invention according to the first aspect, a liquid composition containing two or more kinds of the sugar alcohol at a predetermined ratio is utilized.
That is, by adjusting to the sugar composition to specific one, an eutectic crystalline sugar alcohol that represents a single melting peak obtained by differential scanning calorimetry can be produced. When it is produced using any sugar composition other than specific one, two or more melting peaks can be represented by differential scanning calorimetry. It means that the characteristics of the present invention, such as good feeling of dissolving in the mouth, excellent quality of sweetness, low hygroscopicity, and excellent grindability cannot be attained.

It is difficult to principally define the specific sugar composition since it may vary depending on a kind of the sugar alcohol to be used. For instance, for the production of an eutectic crystalline sorbitol/maltitol, the content of maltitol is 49 to 20% by weight with respect to 51 to 80% by weight of sorbitol, preferably the content of maltitol is 45 to 25% by weight with respect to 55 to 70% by weight of sorbitol.
In addition, for instance, when an eutectic crystalline erythritol/maltitol is produced, the content of maltitol is 70 to 20% by weight with respect to 30 to 80% by weight of erythritol, preferably the content of maltitol is 60 to 40% by weight with respect to 40 to 60% by weight of erythritol.
In those cases, in addition to the sorbitol or both the erythritol and the maltitol, any other kind of the sugar alcohol, such as maltooligosaccharide alcohol, may be included in an amount of less than 10% by weight, preferably in an amount of less than 8% by weight.
On kneading, the liquid composition containing two or more kinds of the sugar alcohol at a predetermined ratio may be one prepared by condensing a liquid product of the sugar alcohol to lower the moisture content thereof. Alternatively, it may be a molten product prepared by melting a powdery product of the sugar alcohol by heat. The condensation may be preliminary carried out so as to have moisture content of 5% or less, preferably 3% or less, more preferably 1% or less. Therefore, the crystallization can be more facilitated and the subsequent drying step can be thus omitted or efficiently carried out.

In the present invention according to the first aspect, therefore, a powder containing crystals of two or more kinds of the sugar alcohol at substantially the same usage ratio as that of the sugar alcohol is added to the liquid composition containing the same two or more kinds of the sugar alcohol at a predetermined ratio, followed by kneading.
For example, when a liquid composition containing both sorbitol and maltitol as sugar alcohol is used, a powder containing crystals of the same sorbitol and maltitol as seed crystals may be used. The powder containing both the sorbitol and maltitol crystals may be a mixture of both the sorbitol and maltitol crystals or an eutectic crystalline sorbitol/maltitol.
The addition of the above liquid composition containing two or more kinds of the sugar alcohol at a predetermined ratio must be a powder containing crystals of the same two or more kinds of the sugar alcohol at substantially the same usage ratio as that of the sugar alcohol. For instance, when another kind of the sugar alcohol, which is different from any one of those described above, or even the same crystals of two or more kinds of the sugar alcohol as those described above is used, the aimed eutectic crystalline sugar alcohol cannot be obtained if the powder having a usage ratio substantially different from that of the sugar alcohol is used.

Here, the substantially the same usage ratio as that of the sugar alcohol does not mean that it should be always the same as the usage ratio of the sugar alcohol but substantially within the range of being thought to be similar. In other words, the usage ratio of each of sugar alcohol components corresponds to the usage ratio of each kind of the sugar alcohol in the seed crystal with an accuracy of ±20% by weight, preferably ±10% by weight. For example, it means that if the usage ratio between two kinds of the sugar alcohol (referred to as the former and the latter) is that the weight ratio of the former to the latter is 60 to 40, the powder to be used contains sugar alcohol crystals as seed crystals such that the weight ratio between the former and the latter is 80 : 20 to 40 : 60, preferably 70 : 30 to 50 : 50.

Specifically, in the present invention according to the first aspect, the eutectic crystalline sugar alcohol of the present invention can be produced by condensing the liquid composition containing two or more kinds of the sugar alcohol at a predetermined ratio under the reduced pressure or thermally melting powders of two or more kinds of the sugar alcohol to make the liquid composition to have a moisture content of about 5% or less, adding a powder that contains the same crystals of two or more kinds of the sugar alcohol (seed crystals) as those described above at a ratio substantially same as the usage ratio of the sugar alcohol to the liquid composition, followed by kneading and aging to co-crystallize the sugar alcohol, and optionally pulverizing the eutectic product.

Here, the total loading amount of the powder containing crystals of two or more kinds of the sugar alcohol to be used as seed crystals (crystal species) is comparatively small as 1 to 35% by weight, preferably 5 to 30% by weight of the usage amount of the liquid composition containing two or more kinds of the sugar alcohol at a predetermined ratio (but, previously condensed so as to have moisture content of 1% or less). As the liquid composition containing two or more kinds of the sugar alcohol at a predetermined ratio is configured so as to be facilitated in crystallization, an increase in yield may be caused to permit a reduction in seed crystals (crystal species).
Furthermore, the total loading amount of the powder containing crystals of two or more kinds of the sugar alcohol to be used as seed crystals (crystal species) may slightly vary depending on the kind of the sugar alcohol to be used.
For instance, for the production of an eutectic crystalline sorbitol/maltitol, the total loading amount of the powder containing crystals of two or more kinds of the sugar alcohol to be used as seed crystals (crystal species) is 5 to 35% by weight, preferably 10 to 30% by weight of the usage amount of the liquid composition containing two or more kinds of the sugar alcohol at a predetermined ratio (but, previously condensed so as to have moisture content of 1% or less).
On the other hand, for the production of an eutectic crystalline erythritol/maltitol, a sufficient total loading amount of the powder containing crystals of two or more kinds of the sugar alcohol to be used as seed crystals (crystal species) may be as small as 1 to 30% by weight, preferably 5 to 20% by weight of the usage amount of the liquid composition containing two or more kinds of the sugar alcohol at a predetermined ratio (but, previously condensed so as to have moisture content of 1% or less).

The conditions for kneading the powder containing crystals of two or more kinds of the sugar alcohol to be used as seed crystals (crystal seeds) added to the liquid composition containing two or more kinds of the sugar alcohol at a predetermined ratio are, but not specifically limited, those in which the kneading may be carried out for 1 to 30 minutes at a temperature of 50 to 100°C, preferably 5 to 20 minutes at a temperature of 60 to 90°C.
In the present invention, as the liquid composition containing two or more kinds of the sugar alcohol at a predetermined ratio is composed so that the liquid composition containing two or more kinds of the sugar alcohol at a predetermined ratio is apt to crystallize. Therefore, it becomes possible to crystallize efficiently at short times. For this reason, furthermore, the time period of aging, which will be described later, can be also shortened.

After the kneading is carried out as described above, aging is performed. The aging can be carried out for a long time but sufficiently for about 0.5 to 24 hours. Therefore, there is no need of carrying out the aging for several days as long as those of the conventional procedure.
The temperature of the aging is, but not specifically limited, preferably equal to or lower than the lowest melting point among those of the powder containing crystals of two or more kinds of the sugar alcohol to be used as seed crystals (crystal species).
For example, the melting point of the sorbitol crystal is 98 °C, the melting point of the maltitol crystal is 150°C, the melting point of the erythritol crystal is 121°C, and the melting point of the xylitol crystal is 95°C.
In this way, the aimed eutectic crystalline sugar alcohol can be produced.
The resulting eutectic crystalline sugar alcohol can be easily pulverized with a pulverizer.

As described above, the eutectic crystalline sugar alcohol that represents a single melting peak obtained by differential scanning calorimetry can be produced.
The term "single melting peak obtained by differential scanning calorimetry" refers to one having a main melting peak with a surface area percentage of 90% ormore, preferably 95% ormore defined obtained by differential scanning calorimetry. In other words, the single melting peak obtained by differential scanning calorimetry represents any additional melting peaks other than the main melting peak, with a surface area percentage of less than 10%, preferably less than 5%. However, the most preferable one is of having no melting peak except the main melting peak.
The eutectic crystalline sugar alcohol thus obtained is efficiently crystallized within a short time and has characteristics of excellent grindability as well as low hygroscopicity, while being hardly consolidated.

The present invention according to the fourth aspect provides an eutectic crystalline sugar alcohol that represents a single melting peak obtained by differential scanning calorimetry.
That is, the present invention according to the fourth aspect is an eutectic crystalline sugar alcohol that represents a single melting peak obtained by differential scanning calorimetry, obtained by a method according to the first aspect, that is: allowing a liquid composition containing two or more kinds of the sugar alcohol at a predetermined ratio to be added with a powder containing crystals of the same two or more kinds of the sugar alcohol at substantially the same usage ratio as that of the sugar alcohol; and kneading and aging a resulting mixture.

Examples of the eutectic crystalline sugar alcohol that shows a single melting peak obtained by such differential scanning calorimetry include an eutectic crystalline sorbitol/maltitol, an eutectic crystalline erythritol/maltitol, an eutectic crystalline xylitol/maltitol, an eutectic crystalline maltitol/lactitol, an eutectic crystalline sorbitol/lactitol, an eutectic crystalline erythritol/lactitol, an eutectic crystalline xylitol/lactitol, an eutectic crystalline erythritol/xylitol, an eutectic crystalline erythritol/sorbitol, an eutectic crystalline sorbitol/maltitol/erythritol, an eutectic crystalline sorbitol/maltitol/lactitol, an eutectic crystalline sorbitol/α-glucopyranosyl-1,6-mannitol/α-glucopyranosyl-1,6-so rbitol, an eutectic crystalline erythritol/α-glucopyranosyl-1,6-mannitol/α-glucopyranosyl-1,6-sorbitol, and an eutectic crystalline xylitol/α-glucopyranosyl-1,6-mannitol/α-glucopyranosyl-1,6-sor bitol.

The eutectic crystalline sugar alcohol of the present invention according to the fourth aspect represents a single melting peak obtained by differential scanning calorimetry and the temperature for the melting peak is lower than that of the melting peak of the contained sugar alcohol.
In other words, the eutectic crystalline sugar alcohol of the present invention according to the fourth aspect is produced using two or more kinds of the sugar alcohol and the temperature for the melting peak is lower than that of the melting peak of each kind of the sugar alcohol.
Furthermore, the eutectic crystalline sugar alcohol of the present invention according to the fourth aspect has characteristics of excellent grindability as well as low hygroscopicity, while being hardly consolidated.

Furthermore, a powder being adjusted to have a particle size of 150 µm or more but less than 350 µm, which is obtained by pulverizing the eutectic crystalline sugar alcohol of the present invention according to the fourth aspect, is excellent in rate of dissolution and has good feeling of dissolving in the mouth in addition to a preferable quality of sweetness with definite feeling, compared with those of a mixture powder obtained by mixing sugar alcohol adjusted to the similar particle size so as to have the similar sugar composition.

The eutectic crystalline sugar alcohol of the present invention according to the fourth aspect can be obtained using two or more kinds of the sugar alcohol. As described in the fifth aspect, however, it is preferable to use at least one or more kinds of disaccharide sugar alcohol as sugar alcohols. The disaccharide sugar alcohol is one described in the description about the second aspect.

The present invention according to the third aspect relates to an eutectic crystalline sorbitol/maltitol out of the eutectic crystalline sugar alcohols, and is a method of manufacturing an eutectic crystalline sorbitol/maltitol that represents a single melting peak at a temperature of 91 ± 5°C obtained by differential scanning calorimetry, comprising: allowing a liquid composition containing sorbitol in an amount of 51 to 80% by weight and maltitol in an amount of 49 to 20% by weight to be added with a powder containing a sorbitol crystal and a maltitol crystal at the same usage ratio as that of the sorbitol and the maltitol; and kneading and aging a resulting mixture.
According to the method of the present invention according to the third aspect, an eutectic crystalline sorbitol/maltitol that represents a single melting peak at 91 ± 5°C obtained by differential scanning calorimetry as described in the sixth aspect can be obtained.
The eutectic crystalline sorbitol/maltitol has characteristics of a good feeling of dissolving in the mouth without a feeling of astringent taste of sorbitol, while having quality of sweetness with a refreshing definite feeling and excellent grindability with low hygroscopicity.
In particular, a preferable eutectic crystalline sorbitol/maltitol is one obtained by adding a powder containing both the sorbitol and maltitol crystals to a liquid composition containing 55 to 70% by weight of sorbitol and 45 to 25% by weight of maltitol, followed by kneading and aging (i.e., an eutectic crystalline sorbitol/maltitol representing a single melting peak at 91 ± 5°C obtained by differential scanning calorimetry). Here, the same conditions of kneading and aging as those described above are used.

The eutectic crystalline sugar alcohol includes an eutectic crystalline sugar alcohol composed of: for example, sorbitol, erythritol, xylitol, or mannitol; and a disaccharide sugar alcohol such as maltitol, lactitol, α-glucopyranosyl-1,6-mannitol, α-glucopyranosyl-1,6-sorbitol, or cellobitol in addition to the eutectic crystalline sorbitol/maltitol as described in the sixth aspect, and a specific example thereof includes an eutectic crystalline erythritol/maltitol.
The eutectic crystalline erythritol/maltitol is obtained by adding a powder containing erythritol and maltitol crystals to a liquid composition containing 30 to 80% by weight of erythritol and 70 to 20% by weight of maltitol, followed by kneading and aging, which represents a single melting peak at 112 ± 5°C obtained by differential scanning calorimetry. The kneading conditions and also the aging conditions are the same as those described above.
The resulting eutectic crystalline erythritol/maltitol has characteristics of a good feeling of dissolving in the mouth, hardly feeling astringent taste of erythritol, while having quality of sweetness with a refreshing definite feeling and excellent grindability with low hygroscopicity.

In addition, a specific example of the eutectic crystalline sugar alcohol includes an eutectic crystalline erythritol/α-glucopyranosyl-1,6-mannitol/α-glucopyranosyl-1,6-sorbitol.
The eutectic crystalline erythritol/α-glucopyranosyl-1,6-mannitol/α-glucopyranosyl-1, 6-sorbitol is obtained by: adding liquid composition containing 20 to 60% by weight of erythritol, 40 to 20% by weight of α-glucopyranosyl-1,6-mannitol, and 40 to 20% by weight of α-glucopyranosyl-1,6-sorbitol with a powder containing erythritol crystal, α-glucopyranosyl-1,6-mannitol crystal, and α-glucopyranosyl-1,6-sorbitol crystal at a ratio substantially similar to the ratio at which the sugar alcohols are used in the liquid composition; and kneading and aging the resultant. The eutectic crystal shows a single melting peak at 110 ± 5°C obtained by differential scanning calorimetry. It should be noted that kneading conditions and aging conditions are similar to those of described above.
The resulting eutectic crystalline erythritol/α-glucopyranosyl-1,6-mannitol/α-glucopyranosyl-1,6-sorbitol has characteristics of a good feeling of dissolving in the mouth, hardly feeling astringent taste of erythritol, while having quality of sweetness with a refreshing definite feeling and excellent grindability with low hygroscopicity.
Furthermore, a specific example of an eutectic crystalline sugar alcohol includes an eutectic crystalline erythritol/lactitol.
The eutectic crystalline erythritol/lactitol can be obtained by allowing the liquid composition containing 20 to 80% by weight of erythritol and 80 to 20% by weight of lactitol to be added with a powder that contains erythritol and lactitol crystals at substantially the same usage ratio as that of the sugar alcohols in the liquid composition, followed by kneading and aging. The resulting eutectic crystalline erythritol/lactitol represents a single melting peak at 113 ± 5°C obtained by differential scanning calorimetry. Here, the same conditions of kneading and aging as those described above are used.
The resulting eutectic crystalline erythritol/lactitol has characteristics of a good feeling of dissolving in the mouth, hardly feeling astringent taste of erythritol, while having quality of sweetness with a refreshing definite feeling and excellent grindability with low hygroscopicity.
Furthermore, a specific example of the eutectic crystalline sugar alcohol includes an eutectic crystalline xylitol/maltitol.
The eutectic crystalline xylitol/maltitol can be obtained by allowing the liquid composition containing 50 to 80% by weight of xylitol and 50 to 20% by weight of maltitol to be added with a powder that contains xylitol and maltitol crystals at substantially the same usage ratio as that of the sugar alcohols in the liquid composition, followed by kneading and aging. The resulting eutectic crystalline xylitol/maltitol represents a single melting peak at 93 ± 5°C obtained by differential scanning calorimetry. Here, the same conditions of kneading and aging as those described above are used.
The resulting eutectic crystalline xylitol/maltitol has characteristics of a good feeling of dissolving in the mouth, hardly feeling astringent taste of xylitol, while having quality of sweetness with a refreshing definite feeling and excellent grindability with low hygroscopicity.
Furthermore, a specific example of the eutectic crystalline sugar alcohol includes an eutectic crystalline erythritol/xylitol.
The eutectic crystalline erythritol/xylitol can be obtained by allowing a liquid composition containing 20 to 50% by weight of erythritol and 80 to 50% by weight of xylitol to be added with a powder that contains erythritol and xylitol crystals at substantially the same usage ratio as that of the sugar alcohols in the liquid composition, followed by kneading and aging. The resulting eutectic crystalline erythritol/xylitol represents a single melting peak at 93 ± 5°C obtained by differential scanning calorimetry. Here, the same conditions of kneading and aging as those described above are used.
The resulting eutectic crystalline erythritol/xylitol has characteristics of a good feeling of dissolving in the mouth, a coolness feeling and a refreshing definite feeling, but hardly feeling astringent taste, as well as excellent grindability with low hygroscopicity.

Furthermore, as described in the seventh aspect, because of improvements on the rate of dissolution, dispersibility, good feeling of dissolving in the mouth, and the like, it is preferable to design the eutectic crystal according to any one of the fourth to sixth aspects to have 95% or more of a powder part having a particle size of less than 350 µm (i.e., less than 5% of a crude particle part having a particle size of 350 µm or more). In the eutectic crystal of the present invention, the eutectic crystal shows the highest rate of dissolution and has a merit of a shortened working time in use when the crystals of the respective kinds of the sugar alcohol and a mixture thereof are compared with each other with respect to their rates of dissolution while making their particle sizes uniform. In other words, the eutectic crystal according to any one of the fourth to sixth aspects can be easily pulverized into fine particles but hardly consolidated even if the particle size of the powder is made smaller to some extent, as well as having excellent dissolving and dispersing abilities, good feeling of dissolving in the mouth, and the like.

The present invention according to the eighth aspect relates to a powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, in which a moisture content is 2% by weight or less.

The term "powder containing an eutectic crystalline sorbitol/maltitol" as used in the present invention according to the eighth aspect refers to a powdery composition containing an eutectic crystalline sorbitol/maltitol, which is obtained by allowing a liquid composition containing 40 to 80% by weight of sorbitol and 60 to 20% by weight of maltitol to be added with a powder containing a sorbitol crystal and a maltitol crystal at the same usage ratio as that of the sorbitol and the maltitol, respectively, followed by kneading and aging, while both the sorbitol and the maltitol in the liquid composition are co-crystallized. In addition, any area in an amorphous or crystalline state of any component such as sorbitol, maltitol, maltotriitol, or maltotetraitol may be contained, other than those in a eutectic state of sorbitol and maltitol. It is noted that the eutectic crystalline sorbitol/maltitol of the present invention does not contain any mixture in which powders of sorbitol and maltitol are simply mixed together. One of examples of the powdery composition is a hydrogenated starch hydrolysate powder disclosed in JP 2002-253167 A. The pulverization of the hydrogenated starch hydrolysate may be carried out using any one of methods known in the art. The hydrogenated starch hydrolysate is generally produced by hydrogenation of a starch hydrolysate obtained by hydrolyzing starch using an acid, an enzyme, or the like. The highly-saccharificated hydrogenated starch hydrolysate refers to one obtained by subjecting a highly-saccharificated starch syrup to hydrogenation. The hydrogenated starch hydrolysate to be used in the present invention may be prepared by any method. For example, the hydrogenated starch hydrolysate may be obtained by subjecting a mixture of two or more different starch hydrolysates and sugars prepared separately to hydrogenation. In addition, the hydrogenated starch hydrolysate may be obtained by mixing two or more kinds of the sugar alcohol and hydrogenated starch hydrolysate prepared separately. Furthermore, the hydrogenated starch hydrolysate may be obtained by subjecting a fractionated product from a prepared starch hydrolysate by means of chromatography or the like to hydrogenation. Furthermore, the hydrogenated starch hydrolysate may be one obtained by fractionating the prepared hydrogenated starch hydrolysate using chromatography or the like.

Furthermore, in JP 2002-253167 A, there is disclosed a method of manufacturing a hydrogenated starch hydrolysate powder, which is characterized by adding a seed crystal (seed) to a hydrogenated starch hydrolysate mainly containing sorbitol and maltitol and then kneading a mixture to allow crystallization of both the sorbitol and the maltitol in the hydrogenated starch hydrolysate. Therefore, in this way, the resulting powder containing an eutectic crystalline sorbitol/maltitol referred by the present invention is a powdery composition mainly containing sorbitol and maltitol in a hydrogenated starch hydrolysate, which are co-crystallized therein.

The powder containing an eutectic crystalline sorbitol/maltitol of the present invention according to the eighth aspect is, as described in the thirteenth aspect, preferably one in which the content of the sorbitol is 40 to 60% by weight and the content of the maltitol is 60 to 40% by weight, while the content of high molecular sugar alcohol, which is not smaller than maltotriitol, is less than 10% by weight. In particular, one in which the content of sorbitol is 40 to 50% by weight, the content of maltitol is 55 to 45% by weight or more, and the content of high molecular sugar alcohol, which is not smaller than maltotriitol, is less than 8% by weight is more preferable.
Here, when the content of high molecular sugar alcohol, which is not smaller than maltotriitol, becomes 10% by weight or more, a problem such as facilitated consolidation may occur.

The present invention according to the eighth aspect is a powdery composition that contains an eutectic crystalline sorbitol/maltitol containing 40 to 80% by weight of sorbitol and 60 to 20% by weight of maltitol and representing a melting peak at 91 ± 5°C obtained by differential scanning calorimetry, while the moisture content thereof (the percentage content of moisture) is 2% by weight or less. A method of adjusting the moisture content (the percentage content of moisture) to 2% by weight or less may be any one of methods known in the art, for example a drying method. Examples of the drying method typically include fluidized-bed drying, vacuum drying, and warm-air drying. In the present invention, any drying method may be used. Another method may be used is pulverization of a hydrogenated starch hydrolysate having a moisture content of 2% by weight or less so as to adjust the moisture content of the powder containing eutectic crystalline sorbitol/maltitol to 2% by weight or less. In addition, the powder containing an eutectic crystalline sorbitol/maltitol may be placed in a packing material together with an absorbent (e.g., silica gel) to adjust the moisture content of the powder containing an eutectic crystalline sorbitol/maltitol in the packing material to 2% by weight or less.
In addition, the consolidation property can be improved when the moisture content is adjusted to 2% by weight or less. A further improvement in consolidation property can be attained when the moisture content is preferably adjusted to 1% by weight or less, more preferably adjusted to 0.5% by weight or less.

Next, the present invention according to the ninth aspect relates to an powder containing an eutectic crystalline sorbitol/maltitol, comprising of a powdery composition that contains an eutectic crystalline sorbitol/maltitol including sorbitol in an amount of 40 to 80% by weight and maltitol in an amount of 60 to 20% by weight and having a melting peak at a temperature of 91 ± 5°C obtained by differential scanning calorimetry, in which the percentage content of a fine powder having a particle size of less than 150 µm is 30% or less.
The present invention according to the ninth aspect is different from the present invention according to the eighth aspect in terms of defining the content of fine powder to have a particle size of less than 150 µm, comparing that the present invention according to the eighth aspect described above defines the moisture content (the percentage content of moisture).

A method of adjusting the content of fine powder having a particle size of less than 150 µm to 30% or less may be any one of methods known in the art. Among them, an exemplified method is classification. The classification method may be generally, for example, air classification or sieve classification. In the present invention, any classification method may be applied. In addition, the method may be a pulverization method in which a powder is pulverized such that the percentage content of a fine powder having a particle size of less than 150 µm becomes 30% or less in the pulverized powder. Alternatively, the method may be a granulating method or the like in which a powder is pulverized such that the percentage content of a fine powder having a particle size of less than 150 µm becomes 30% or less in the pulverized powder.

Furthermore, the powder of carbohydrate can be generally classified into a granulated product that largely contains a crude particle part adjusted to have a particle size of less than 2 mm and a powdery product that mainly contains a fine part adjusted to have a particle size of less than 350 µm.
Here, in the case of the powdery product having a particle size distribution with a major part of a particle size of less than 350 µm, consolidation may be facilitated in comparison with the particle part having a particle size distribution with a major part of a crude particle size of 350 µm or more. But, with such a procedure to improve consolidation as proposed by the present invention, the powder can bear the distribution.

However, as described in the twelfth aspect, in the powder containing an eutectic crystalline sorbitol/maltitol according to any one of the eighth to eleventh aspects, it is preferable that the content of the powdery part having a particle size of less than 350 µm is 95% or more (i.e., the content of the crude part having a particle size of 350 µm or more is less than 5%) because of improvements in rate of dissolution, dispersibility, and also good feeling of dissolving in the mouth. Furthermore, in the case of making a comparison among the powder containing an eutectic crystalline sorbitol/maltitol of the present invention, the sorbitol crystalline, maltitol crystalline, and a mixture of both crystals while making their particle sizes uniform, the powder containing an eutectic crystalline sorbitol/maltitol shows the highest rate of dissolution and has a merit of a shortened working time in use. That is, the powder containing an eutectic crystalline sorbitol/maltitol according to any one of the eighth to eleventh aspects can be easily pulverized into fine particles but hardly consolidated even if the particle size of the powder is made smaller to some extent, as well as having excellent dissolving and dispersing abilities, good feeling of dissolving in the mouth, and the like.

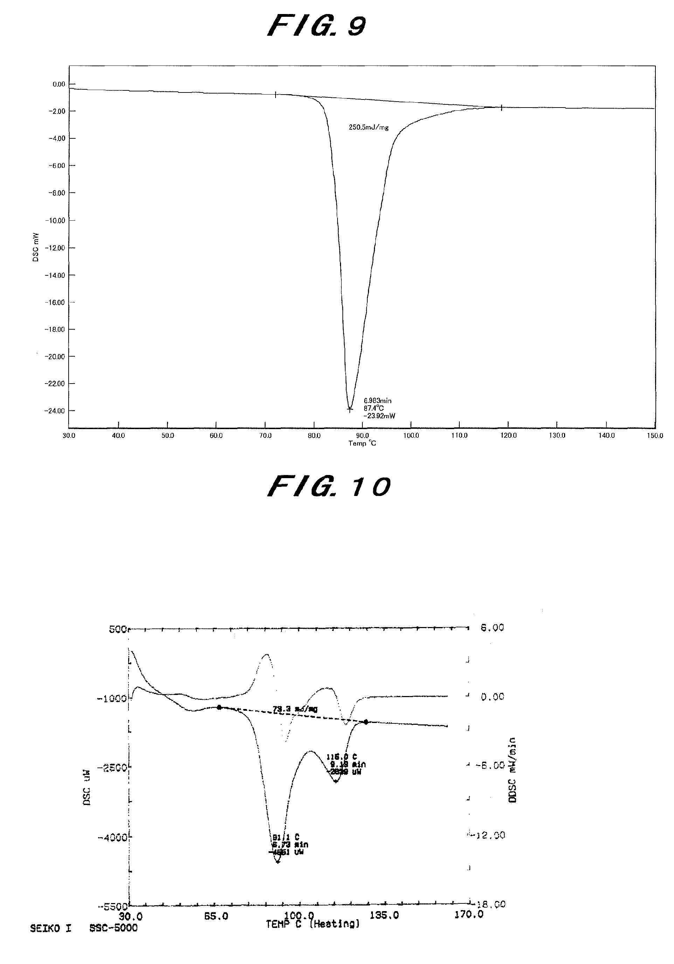

Furthermore, the present invention according to the tenth aspect relates to a powder containing an eutectic crystalline sorbitol/maltitol, comprising a powdery composition that contains an eutectic crystalline sorbitol/maltitol containing 40 to 80% by weight of sorbitol and 60 to 20% by weight of maltitol and representing a melting peak at 91 ± 5°C obtained by differential scanning calorimetry, while having a melting calorie of 70 J/g or more. The present invention according to the tenth aspect is different from the present invention according to the eighth aspect described above in that the present invention according to the eighth aspect defines the moisture content (the percentage content of moisture) while the present invention according to the tenth aspect defines a melting calorie to be 70 J/g or more. As a method of adjusting the melting calorie to 70 J/g or more, any method can be applied. For example, a shearing force in kneading may be increased. Also, a method of increasing the viscosity of a mixture or shearing force may be used, by means of using a two-axis blending machine or an extruder which can apply a strong shearing force, extending a kneading period, increasing rate of seed supply, and decreasing temperature of a kneading product in kneading. In addition, a method of aging, which accelerates the crystallization while keeping the temperature at 20 to 80°C after kneading, may be used. Any one of methods generally used in the art may be applied as a measuring method of melting calories. Among them, a measuring method using differential scanning calorimetry (DSC) may be preferably used because of its simpleness and precision. The powder containing an eutectic crystalline sorbitol/maltitol of the present invention is measured by differential scanning calorimetry. In this case, as shown in Fig. 10, it is characterized by having a main melting peak at approximately 91°C and additional melting peaks may sometimes be also observed. As shown in Fig. 10, when plural melting peaks are generated on a differential heat capacity curve, the melting calorie can be calculated from the total area of all the peaks. When the melting calorie obtained by the method is 70 J/g or more, it becomes possible to improve consolidation and impart excellent non-consolidation properties.

The present invention according to any one of the eighth to tenth aspects exhibits three different methods for an improvement in consolidation: one for adjusting the moisture content to 2% by weight or less; one for adjusting the percentage content of a fine powder having a particle size of less than 150 µm to 30% or less; and one for adjusting the melting calorie to 70 J/g or more. Those methods may be carried out independently or in combination. In addition, by adjusting the content of high molecular sugar alcohol, which is not smaller than maltotriitol, to less than 10% by weight, the consolidation property can be improved to make non-consolidation property excellent.

The present invention according to the eleventh aspect relates to a powder containing an eutectic crystalline sorbitol/maltitol, comprising a powdery composition that contains an eutectic crystalline sorbitol/maltitol containing mainly sorbitol and maltitol, which are co-crystallized, in which a moisture content is 2% by weight or less, and/or a percentage content of fine powder having a particle size of less than 150 µm is 30% or less, and/or a melting calorie is 70J/g or more.
In the present invention according to the eleventh aspect, three requirements respectively defined in the present inventions according to any one of the eighth to tenth aspects are combined using the term "and/or". The present invention according to the eleventh aspect does not include the present inventions according to any one of the eighth to tenth aspects, so that the combination of the above requirements using the term "or" only is omitted from the combinations using the term "and/or".
Therefore, the present invention according to the eleventh aspect specifically includes three combinations of:
(1) 2% by weight or less of the moisture content or 30% or less of the percentage content of fine powder having a particle size of less than 150 µm, and 70 J/g or more of melting calorie;
(2) 2% by weight or less of moisture content, and 30% or less of the percentage content of fine powder having a particle size of less than 150 µm, or 70 J/g or more of melting calorie; and
(3) 2% by weight or less of moisture content, and 30% or less of the percentage content of fine powder having a particle size of 150 µm or less, and 70 J/g or more of melting calorie.

The powder containing an eutectic crystalline sorbitol/maltitol obtained by the method of the present invention according to any one of the eighth to thirteenth aspects may be packed in any packaging configuration such as a corrugated cardboard packaging, a craft packaging, a flexible container bag, or a container. For preventing the consolidation, the corrugated cardboard packaging is preferable.

The present invention according to the fourteenth aspect relates to a method of manufacturing a composition containing an eutectic crystalline sugar alcohol, comprising: an eutectic crystalline sugar alcohol that represents a single melting peak obtained by differential scanning calorimetry; and ingredients of food products, food additives, pharmaceutical excipients, pharmaceutical products, or cosmetic products. In the method, ingredients of food products, food additives, pharmaceutical excipients, pharmaceutical products, or cosmetic products is added to the liquid composition containing two or more kinds of the sugar alcohol at a predetermined ratio, and then a powder containing crystals of the same two or more kinds of the sugar alcohol at substantially the same usage ratio as that of the sugar alcohol is added to the mixture, followed kneading and aging. Alternatively, ingredients of food products, food additives, pharmaceutical excipients, pharmaceutical products, or cosmetic products may be added when a liquid composition containing two or more kinds of the sugar alcohol at a predetermined ratio is added with a powder containing crystals of the same two or more kinds of the sugar alcohol at substantially the same usage ratio as that of the sugar alcohol, followed by kneading and aging.

In other words, the present invention according to the fourteenth aspect relates to a method of manufacturing a composition containing an eutectic crystalline sugar alcohol that contains: an eutectic crystalline sugar alcohol that represents a single melting peak obtained by differential scanning calorimetry; and ingredients of food products, food additives, pharmaceutical excipients, pharmaceutical products, or cosmetic products. In the production of the eutectic crystalline sugar alcohol by the method of the present invention according to the first aspect, ingredients of food products food additives, pharmaceutical excipients, pharmaceutical products, or cosmetic products is added, whereby producing the composition containing an eutectic crystalline sugar alcohol, which contains: a co-crystallized sugar alcohol, which contains: an eutectic crystalline sugar alcohol that represents a single melting peak obtained by differential scanning calorimetry; and ingredients of food products, food additives, pharmaceutical excipients, pharmaceutical products, or cosmetic products.
The addition of ingredients of food products, food additives, pharmaceutical excipients, pharmaceutical products, or cosmetic products may be carried out in at any time as described above.
According to the present invention according to the fourteenth aspect, a composition containing an eutectic crystalline sugar alcohol, which contains an eutectic crystalline sugar alcohol according to the sixteenth aspect and ingredients of food products, food additives, pharmaceutical excipients, pharmaceutical products, or cosmetic products, can be obtained.

The present invention according to the fifteenth aspect relates to a method of manufacturing a powdery composition, comprising a powder containing an eutectic crystalline sorbitol/maltitol representing a melting peak of 91 ± 5°C obtained by differential scanning calorimetry, and ingredients of food products, food additives, pharmaceutical excipients, pharmaceutical products, or cosmetic products. The method is characterized by adding ingredients of food products, food additives, pharmaceutical excipients, pharmaceutical products, or cosmetic products to a liquid composition containing 40 to 80% by weight of sorbitol and 60 to 20% by weight or more of maltitol, followed by addition of a powder containing crystals of sorbitol and maltitol at substantially the same usage ratio as that of sorbitol and maltitol, and kneading and aging a resulting mixture; or adding a powder containing crystals of sorbitol and maltitol at substantially the same usage ratio as that of sorbitol and maltitol to a liquid composition containing 40 to 80% by weight of sorbitol and 60 to 20% by weight or more of maltitol, simultaneously with addition of ingredients of food products, food additives, pharmaceutical excipients, pharmaceutical products, or cosmetic products, followed by kneading and aging.

That is, the present invention according to the fifteenth aspect relates to a method of manufacturing a powdery composition, comprising a powder containing an eutectic crystalline sorbitol/maltitol representing a melting peak at 91 ± 5°C obtained by differential scanning calorimetry, and ingredients of food products, food additives pharmaceutical excipients, pharmaceutical products, or cosmetic products. In the production of an eutectic crystalline sugar alcohol by the method of the present invention according to the third aspect, ingredients of food products, food additives, pharmaceutical excipients, pharmaceutical products, or cosmetic products is added, so that a powdery composition can be produced comprising a powder containing an eutectic crystalline sorbitol/maltitol representing a melting peak at 91 ± 5°C obtained by differential scanning calorimetry and ingredients of food products, food additives, pharmaceutical excipients, pharmaceutical products, or cosmetic products.
The addition of ingredients of food products, food additives, pharmaceutical excipients, pharmaceutical products, or cosmetic products may be carried out at any time as described above.
According to the present invention according to the fifteenth aspect, a powdery composition containing: a powder containing an eutectic crystalline sorbitol/maltitol described in the seventeenth aspect; and ingredients of food products, food additives, pharmaceutical excipients, pharmaceutical products, or cosmetic products can be obtained.

Here, the ingredients of food products, food additives, pharmaceutical excipients, pharmaceutical products, and cosmetic products may be, but not limited as far as those generally used, one or two or more kinds selected from a high-intensity sweetener, oligosaccharide, dietary fiber, saccharides, sugar alcohols, acidulant, vitamins, minerals, coloring agents, flavor, and functional ingredients.
Examples of the high-intensity sweetener include: aspartame, acesulfame K, sucralose, saccharin and salts thereof; stevioside and glycosides thereof; glycyrrhizin and salts thereof; thaumatin; neotame; alitame; and neohesperidin dihydrochalcone.
Examples of the oligosaccharide include fructooligosaccharide, galactooligosaccharide, lactulose, raffinose, soybean oligosaccharide, isomaltooligosaccharide, maltooligosaccharide, xylooligosaccharide, cellooligosaccharide, agarooligosaccharide, chitinoligosaccharide, chitosanoligosaccharide, and cyclodextrin.
Examples of the dietary fiber include polydextrose, indigestible dextrin, cellulose, hemicellulose, glucomannan, apple fiber, corn fiber, beet fiber, pectin, chitin, and chitosan.

Examples of the saccharides include sugar, fructose, galactose, arabinose, xylose, glucose, mannose, lactose, palatinose, trehalose, glucosamine, and N-acetyl glucosamine.
Examples of the sugar alcohols include sorbitol, maltitol, erythritol, xylitol, mannitol, inositol, lactitol, α-glucopyranosyl-1,6-mannitol (α-glucopyranosyl-1,1-mannitol), α-glucopyranosyl-1,6-sorbitol, cellobitol, and maltooligosaccharide alcohols such as maltotriitol and maltotetraitol.
Examples of the acidulant include citric acid, malic acid, acetic acid, lactic acid, and gluconic acid.
Examples of the vitamins include vitamin C, vitamin B1, vitamin B2, vitamin B6, vitamin B12, folic acid, pantothenic acid, biotin, niacin, nicotinic acid amide, vitamin A, vitamin D, and vitamin E.
The minerals may be either an inorganic salt or organic salt, and the minerals may be any one of mineral-enriched yeast, a natural product and an extract thereof rich in minerals. Examples of the iron that can be used include sodium ferrous citrate, ferrous citrate, ammonium ferric citrate, ferrous pyrophosphate, ferric pyrophosphate, ferrous gluconate, iron lactate, hemoferrum, ferrous sulfate, ferric sulfate, and ferric chloride. Examples of the zinc that can be used include zinc sulfate and zinc gluconate, and examples of the copper that can be used include copper sulfate and copper gluconate. Examples of the calcium that can be used include calcium citrate, calcium gluconate, calcium lactate, calcium monohydrogen phosphate, tricalcium phosphate, calcium dihydrogen phosphate, calcium carbonate, calcium chloride, calcium dihydrogen pyrophosphate, calcium hydroxide, calcium pantothenate, calcium propionate, calcium sulfate, calcium eggshell, fish bone powder, whey calcium, petrified seaweed, coral powder, pearl oyster, scallop, and oystershell. Examples of the magnesium that can be used include magnesium chloride, magnesium sulfate, magnesium carbonate, and magnesium phosphate. Examples of the phosphorus that can be used include potassium phosphate, calcium phosphate, and sodium phosphate. Additionally, mineral-enriched yeast, beer yeast, dolomite, bittern, deep ocean water, kelp mineral, or the like can be used as a natural product material.
The coloring agents include a natural pigment and a synthetic coloring agent. Examples of the coloring agents include a paprika color, a monascus color, a cochineal color, a lac color, a beet red color, an anthocyanin-based color, an annatto color, a gardenia yellow color, a gardenia blue color, a gardenia red color, a carthamus color, a caramel color, a carotenoid-based color, a turmeric color, a kaoliang color, a madder color, a spirulina color, and synthetic coloring agents thereof.
The flavor includes various natural flavors and synthetic flavors. Examples of the functional ingredient include polyphenols, carnitine, coenzyme Q10, isoflavone, soybean protein, soybean globulin, caseinphosphopeptide, amino acid, peptides, various plant extracts, and other pharmaceutical properties or crude drug ingredients.
The present invention can also provide a composition excellent in uniformity, even when a trace amount of an ingredient is added thereto.

The content of ingredients of food products, food additives, pharmaceutical excipients, pharmaceutical products, or cosmetic products in the composition of the present invention according to the sixteenth or seventeenth aspect is not particularly limited as far as it is suitably selected on the basis of an intended purpose. In general, however, the content of the ingredient is 0.01 to 50 parts by weight, preferably 0.05 to 20 parts by weight with respect to 100 parts by weight of the eutectic crystalline sugar alcohol.

### Examples

Hereinafter, the present invention will be described more specifically with reference to the examples and the like of the present invention. However, the present invention does not intend to be restricted by those examples.

### Example 1 (Production of eutectic crystalline sorbitol/maltitol)

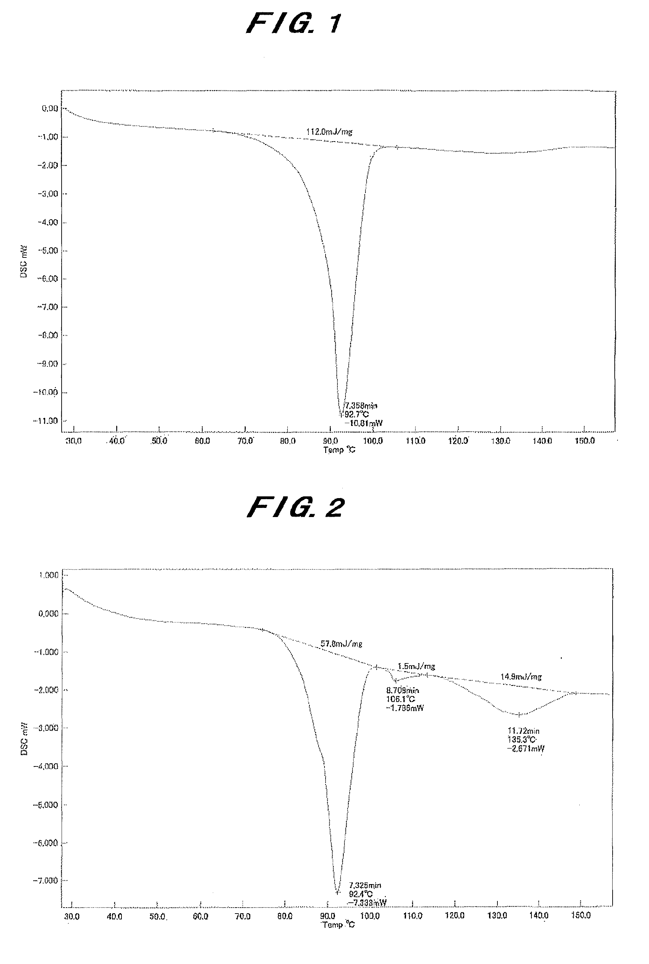

Seed crystals including 14 g of sorbitol and 6 g of maltitol were added to 100 g of a viscous liquid composition obtained by condensing an aqueous sugar alcohol solution containing 67% of sorbitol, 29% of maltitol, and 4% of maltooligosaccharide alcohol of not smaller than maltotriitol to a moisture concentration of 1% or less under reduced pressure, followed by kneading at 80°C for about 15 minutes. Subsequently, the mixture was stored in a thermostatic chamber at 50°C to carry out aging. After 12-hour aging, the mixture was pulverized with a hummer type pulverizer. Consequently, 110 g of powder (eutectic crystalline sorbitol/maltitol) was obtained without causing any clogging or the like in the pulverizer.
The sugar composition of the aqueous sugar alcohol solution and the composition of the seed crystal are shown in Table 1.
The resulting powder had fluidity and a weight growth rate of less than 3% even after storing for one week at 25°C with a relative humidity of 52%.
The resulting powder was analyzed using differential scanning calorimetry (DSC). As a result, a single melting peak was observed at 92.7°C. In Fig. 1, the resulting DSC curve of the powder (eutectic crystalline sorbitol/maltitol) is illustrated.

### Comparative Example 1

A powder was obtained by the same way as that of Example 1, except that the sugar composition of an aqueous sugar alcohol solution was adjusted as shown in Table 1 and a seed crystal was added at a ratio shown in Table 1. The resulting DSC curve of the resulting powder is shown in Fig. 2.

### Example 2

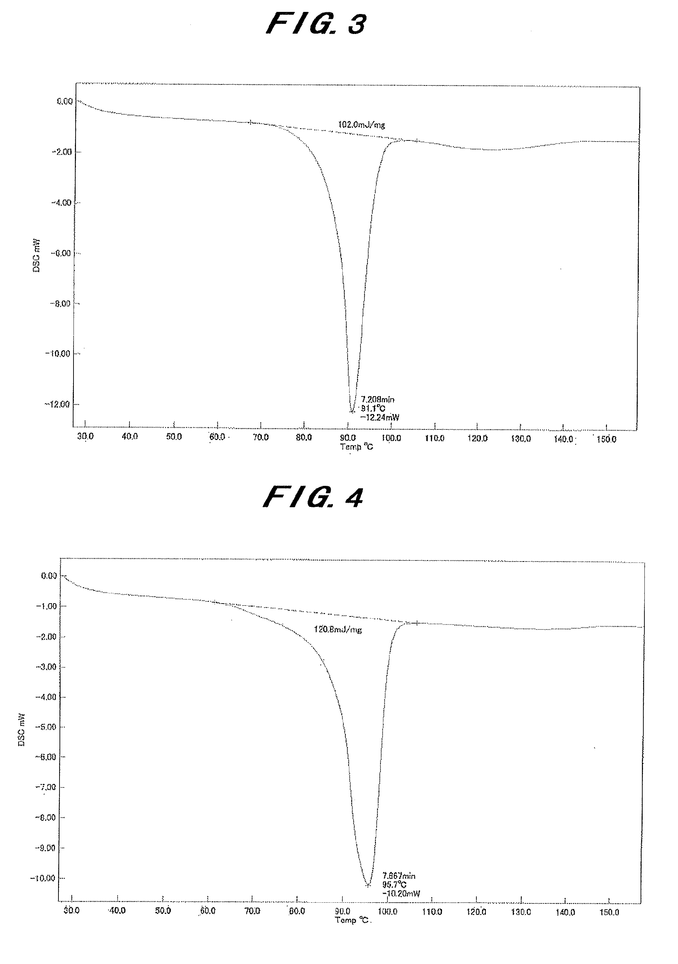

A powder was obtained by the same way as that of Example 1, except that the sugar composition of an aqueous sugar alcohol solution was adjusted as shown in Table 1 and a seed crystal was added at a ratio shown in Table 1. The resulting DSC curve of the resulting powder (eutectic crystalline sorbitol/maltitol) is shown in Fig. 3.

### Example 3

A powder was obtained by the same way as that of Example 1, except that the sugar composition of an aqueous sugar alcohol solution was adjusted as shown in Table 1 and a seed crystal was added at a ratio shown in Table 1. The resulting DSC curve of the resulting powder (eutectic crystalline sorbitol/maltitol) is shown in Fig. 4.

**[Table 1]**

| | Sugar composition of aqueous sugar alcohol solution | | | Composition of seed crystal | |
|---|---|---|---|---|---|
| | Sorbitol | Maltitol | Maltooligo sugar alcohol | Sorbitol | Maltitol |
| Example 1 | 67% | 29% | 4% | 14g | 6g |
| Comparative Example 1 | 48% | 48% | 4% | 10g | 10g |
| Example 2 | 57% | 39% | 4% | 12g | 8g |
| Example 3 | 77% | 19% | 4% | 16g | 4g |

The DSC analysis resulted in a completely 100% single melting peak at 91.1°C in Example 2 and at 95.7°C in Example 3.
On the other hand, in Comparative Example 1, a main melting peak at 92.4°C was confirmed and also small peaks at 106.1°C and 135.3°C were confirmed, respectively. The surface percentage of the main melting peak was 78%.
Furthermore, the eutectic crystal thus obtainedwas pulverized with a hammer type pulverizer. The eutectic crystal obtained in each of Examples 2 and 3 was able to be pulverized without causing clogging in the pulverizer. However, the eutectic crystal obtained in Comparative Example 1 caused clogging in the pulverizer, so that an efficient pulverization was not able to be attained.
The powder obtained in each of Examples 2 and 3 showed a weight growth rate of less than 3% even after storing for one week at 25°C with a comparative humidity of 52%. In contrast, the powder obtained in Comparative Example 1 showed a weight growth rate of 3% or more.

### Example 4 (Production of eutectic crystalline sorbitol/maltitol using eutectic crystalline sorbitol/maltitol as seed crystal)

100 g of an aqueous sugar alcohol solution containing 67% of sorbitol, 29% of maltitol, and 4% of maltooligosaccharide alcohol of not smaller than maltotriitol, which were used in Example 1, and 20 g of the powder prepared in Example 1 (eutectic crystalline sorbitol/maltitol) used as a seed crystal were used and other conditions used were the same as those used in Example 1.
Consequently, even in the case of using the eutectic crystalline sorbitol/maltitol as a seed crystal, the production of the eutectic crystalline sorbitol/maltitol was able to be attained just as in the case with Example 1.

### Example 5 (Production of eutectic crystalline erythritol/maltitol)

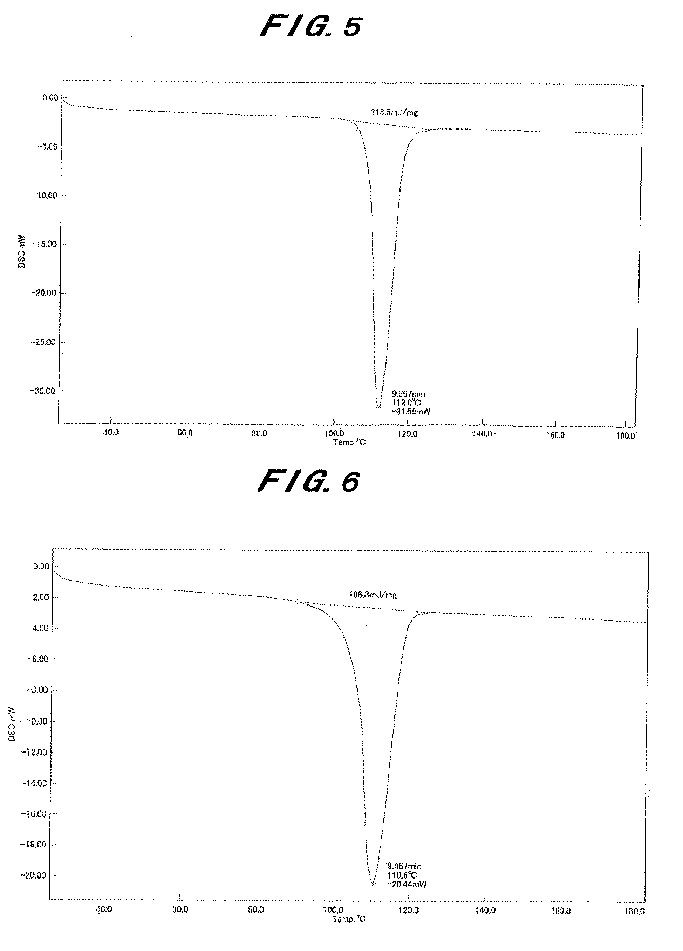

To 100 g of a viscous liquid composition prepared by thermally melting 50 g of erythritol and 50 g of maltitol, 5 g of erythritol and 5 g of maltitol were added as seed crystals, followed by kneading at 80°C for about 10 minutes and storing in a thermostatic chamber at 50°C to carry out aging. After aging for 12 hours, the mixture was pulverized with a hummer type pulverizer, thereby yielding 107 g of powder (eutectic crystalline erythritol/maltitol) without clogging the pulverizer.
The resulting powder was analyzed using differential scanning calorimetry (DSC). The DSC analysis resulted in a completely 100% single melting peak at 112.0°C. The DSC curve of the resulting powder (eutectic crystalline erythritol/maltitol) is shown in Fig. 5.

### Example 6 (eutectic crystalline erythritol/α-glucopyranosyl-1,6-mannitol/α-glucopyranocyl-1,6-sorbitol)

To 100 g of a viscous liquid composition prepared by thermally melting 50 g of erythritol, 25 g of α-glucopyranosyl-1,6-mannitol, and 25 g of α-glucopyranosyl-1,6-sorbitol, 5 g of erythritol, 2.5 g of α-glucopyranosyl-1,6-mannitol, and 2.5 g of α-glucopyranosyl-1, 6-sorbitol were added as seed crystals, followed by kneading at 80°C for about 10 minutes and storing in a thermostatic chamber at 50°C to carry out aging. After aging for 12 hours, the mixture was pulverized with a hummer type pulverizer, thereby yielding 107 g of powder (eutectic crystalline erythritol/α-glucopyranosyl-1,6-mannitol/α-glucopyranosyl-1,6-sorbitol) without clogging the pulverizer.
The resulting powder was analyzed using differential scanning calorimetry (DSC). The DSC analysis resulted in a completely 100% single melting peak at 110.6°C. The DSC curve of the resulting powder is shown in Fig. 6.

### Example 7 (Production of eutectic crystalline erythritol/lactitol)

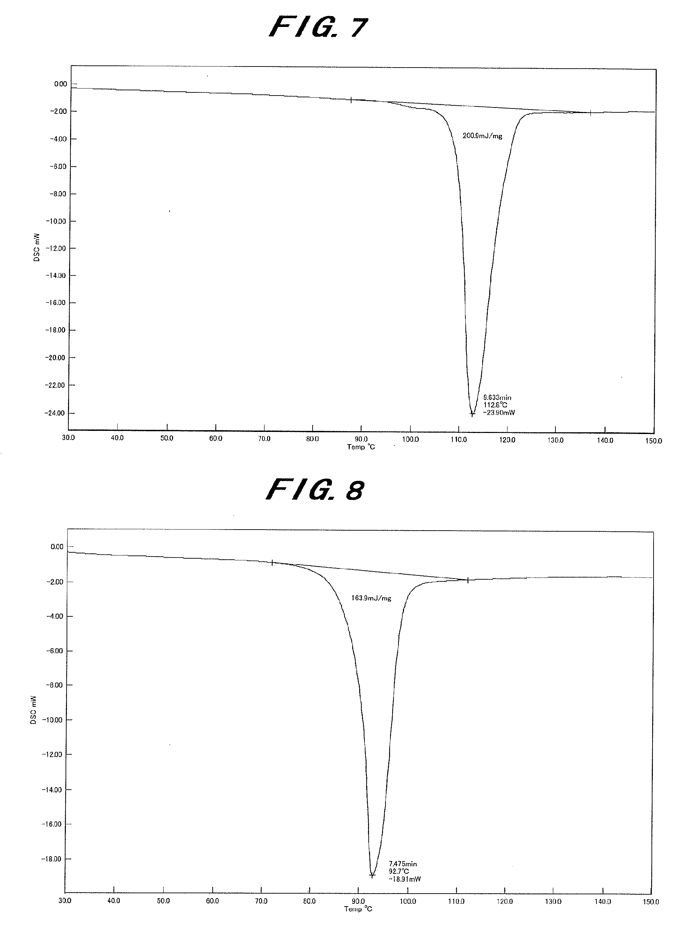

To 100 g of a viscous liquid composition prepared by thermally melting 50 g of erythritol and 50 g of a lactitol monohydrate crystal, 10 g of erythritol and 10 g of a lactitol monohydrate crystal were added as seed crystals, followed by kneading at 90°C for about 20 minutes and storing in a thermostatic chamber at 50°C to carry out aging. After aging for 12 hours, the mixture was pulverized with a hummer type pulverizer, thereby yielding 112 g of powder (eutectic crystalline erythritol/lactitol) without clogging the pulverizer.
The resulting powder was analyzed using differential scanning calorimetry (DSC). The DSC analysis resulted in a completely 100% single melting peak at 112.8°C. The DSC curve of the resulting powder is shown in Fig. 7.

### Example 8 (Production of eutectic crystalline xylitol/maltitol)

To 100 g of a viscous liquid composition prepared by thermally melting 70 g of xylitol and 30 g of maltitol, 21 g of xylitol and 9 g of maltitol were added as seed crystals, followed by kneading at 75°C for about 30 minutes and storing in a thermostatic chamber at 50°C to carry out aging. After aging for 12 hours, the mixture was pulverized with a hummer type pulverizer, thereby yielding 122 gof powder (eutectic crystalline xylitol/maltitol) without clogging the pulverizer.
The resulting powder was analyzed using differential scanning calorimetry (DSC). The DSC analysis resulted in a completely 100% single melting peak at 92.7°C. The DSC curve of the resulting powder is shown in Fig. 8.

### Example 9 (Production of eutectic crystalline erythritol/xylitol)

To 100 g of a viscous liquid composition prepared by thermally melting 30 g of erythritol and 70 g of xylitol, 9 g of erythritol and 21 g of xylitol were added as seed crystals, followed by kneading at 75°C for about 30 minutes and storing in a thermostatic chamber at 50°C to carry out aging. After aging for 12 hours, the mixture was pulverized with a hummer type pulverizer, thereby yielding 123 g of powder (eutectic crystalline erythritol/xylitol) without clogging the pulverizer.
The resulting powder was analyzed using differential scanning calorimetry (DSC). The DSC analysis resulted in a completely 100% single melting peak at 87.4°C. The DSC curve of the resulting powder is shown in Fig. 9.

### Example 10 (Comparison of qualities of sweetness)

The eutectic crystalline sorbitol/maltitol obtained in Example 1 was pulverized to obtain a resulting powder adjusted to have a particle size of 150 µm or more but less than 350 µm. Meanwhile, a mixture powder of sorbitol and maltitol in a ratio of 7 : 3 being adjusted to have a particle size of 150 µm or more but less than 350 µm was prepared. Then, the qualities of sweetness of the two powders were compared as follows.
The powder of eutectic crystalline sorbitol/maltitol was compared with the mixture powder with respect to whether good feeling of dissolving in the mouth, definite feeling, cooling feeling, feeling of astringent taste and overall favorable feeling could be obtained. For each item, the best evaluation was represented by +2 and the worst evaluation was represented by -2. The evaluation was conducted by an organoleptic evaluation with seven panelist by means of a five-stage evaluation (-2, -1, 0, +1, and +2). The results are shown in Table 2.

**[Table 2]**

| | eutectic crystalline Sorbitol/Maltitol |
|---|---|
| Good feeling of dissolving in the mouth | 1.6 |
| Definite feeling | 1.4 |
| No cooling feeling | 0.6 |
| No feeling of astringent taste | 0.9 |
| Overall favorable feeling | 1.6 |

As shown in Table 2, the eutectic crystalline sorbitol/maltitol showed good feeling of dissolving in the mouth and quality of sweetness with definite feeling, while hardly causing a cooling feeling peculiar to sorbitol and unfavorable feeling of astringent taste, compared with the mixture powder of sorbitol and maltitol. Consequently, it showed an overall favorable quality of sweetness.

### Example 11 (Evaluation on non-consolidation property with respect to granulated product)

Hydrogenated starch hydrolysate having a sugar composition containing 44% of sorbitol, 50% of maltitol, and 4% of maltotriitol, and 2% of a high molecular part of not smaller than maltotetraitol was condensed to 97% and then adjusted at 80°C, followed by kneading by adding seed so as using a continuous kneader to adjust the percentage of the seed added to 25% by weight. The powder containing an eutectic crystalline sorbitol/maltitol prepared by the method described in Example 3 of JP 2002-253167 A was used as the seed. After kneading, the powder containing an eutectic crystalline sorbitol/maltitol was left standing for 24 hours at 60°C in a thermostatic chamber. Aftercooling, itwaspulverizedwithahummer mill and then classified to obtain the powder of all less than 2 mm in particle size (granulated product). Fine powders (less than 150 µm in particle size) of the powder containing an eutectic crystalline sorbitol/maltitol each having a moisture content of 0.5% by weight, 1.0% by weight, 2.0% by weight, and 3.0% by weight were adjusted to 20%, 30%, and 40%, respectively. Then, 20 kg of each powder was measured and then placed and sealed in a plastic bag. Subsequently, the plastic bag filled with the powder was packaged in a corrugated board box. Each of the boxes was periodically opened to confirm the consolidation state of the powders.
The consolidation state (non-consolidation property) was evaluated based on the following four stages since in the case of the granulated product being classified such that all of the particles were less than 2 mm in particle size, it is required not to be consolidated for at least about one month, particularly preferably three months or more.
⊚ : No consolidation was observed for three months
○ : No consolidation was observed for two months
□ : No consolidation was observed for one month
× : Consolidation occurred within less than one month

As shown in Table 3, in the case of the granulated product classified so as to have particle size of all less than 2 mm, it was found that no consolidation occur for at least one month when the moisture content is 2.0% by weight or less or when the content of the fine powder having less than 150 µm in particle size is 30% or less. Subsequently, in the case where a granulated product has a moisture content of 2.0% or less and the fine powder of less than 150 µm in particle size is 30% or less, it was found that no consolidation was observed at least for two months.
Furthermore, whenever the moisture content was 0. 5% by weight or less, it was found that no consolidation was observed at least for two months. Furthermore, it was kept examined with respect to the consolidation state. In this case, a granulated product having 30% or less of the fine powder of less than 150 µm in particle size with a moisture content of 1.0% by weight or less showed no consolidation even after three month.

**[Table 3]**

| Evaluation on non-consolidation property of granulated product | | | | |
|---|---|---|---|---|
| | | Content of fine powder having a particle size of less than 150 µm | | |
| | | 20% | 30% | 40% |
| Moisture content | 0.5% by weight | ⊚ | ⊚ | ○ |
| | 1.0% by weight | ⊚ | ⊚ | □ |
| | 2.0% by weight | ○ | ○ | □ |
| | 3.0% by weight | □ | □ | × |

### Example 12 (Evaluation on non-consolidation property of granulated product)

By the same method as that of Example 11, the powder containing an eutectic crystalline sorbitol/maltitol powdered so as to have the addition rate of seed of 25% by weight and 35% by weight was pulverized with a hammer mill and then classified to be adjusted to all less than 2 mm in particle size (granulated product). Subsequently, the powder was adjusted to have 2.0% by weight of the moisture content, followed by being subjected to measurement with differential scanning calorimetry. The melting calorie of the powder containing an eutectic crystalline sorbitol/maltitol was such that the product added with 25% by weight of the seed was 66.3 J/G and the product added with 35% by weight of the seed was 71.2 J/G. Fine powders (less than 150 µm in particle size) of the powder containing an eutectic crystalline sorbitol/maltitol were adjusted to 20%, 30%, and 40%, respectively. Then, 20 kg of each powder was measured and then placed and sealed in a plastic bag. Subsequently, the plastic bag filled with the powder was packaged in a corrugated board box. Each of the boxes was periodically opened to confirm the consolidation state of the powders.
The consolidation state (non-consolidation property) was evaluated based on the following four stages.
⊚ : No consolidation was observed for three months
○ : No consolidation was observed for two months
□ : No consolidation was observed for one month
× : Consolidation occurred within less than one month

As shown in Table 4, when the granulated product was classified to all less than 2 mm in particle size, it was confirmed that the powder containing an eutectic crystalline sorbitol/maltitol having a melting calorie of 70 J/G or more was hardly consolidated, compared with that having a melting calorie of less than 70 J/G. Furthermore, when the content of fine powder having a melting calorie of 70 J/G or more and a particle size of less than 150 µm was adjusted to 30% or less, no consolidation was observed even after three months. Furthermore, the powder containing an eutectic crystalline sorbitol/maltitol, in which no consolidation was observed after three months, was further subjected to the evaluation on consolidation after six months. However, no consolidation was confirmed.
Consequently, with respect to the granulated product, by adjusting the powder containing an eutectic crystalline sorbitol/maltitol to 70 J/G or more in melting calorie, it was confirmed that the product was hardly consolidated.

**[Table 4]**

| Evaluation on non-consolidation property of granulated product | | | | |
|---|---|---|---|---|
| | | Content of fine powder having a particle size of less than 150 µm | | |
| | | 20% | 30% | 40% |
| Melting calorie | 171.2 J/G | ⊚ | ⊚ | ○ |
| | 66.3 J/G | ○ | ○ | □ |

### Example 13 (Evaluation on non-consolidation property of powdery product having particle size of less than 350 µm)

Powder containing an eutectic crystalline sorbitol/maltitol were prepared by the same way as that of Example 11 and then pulverized with a hammer mill, followed by classification, respectively. Therefore, powders all having a particle size of less than 350 µm (powdery products having a particle size of less than 350 µm) were obtained. Subsequently, fine powders (less than 150 µm in particle size) of the powder containing an eutectic crystalline sorbitol/maltitol each having a moisture content of 0.5% by weight, 1.0% by weight, 2.0% by weight, and 3.0% by weight were adjusted to 20%, 30%, and 40%, respectively. Then, 20 kg of each powder was measured and then placed and sealed in a plastic bag. Subsequently, the plastic bag filled with the powder was packaged in a corrugated board box. Each of the boxes was periodically opened to confirm the consolidation state of the powders.
The consolidation state (non-consolidation property) was evaluated based on the following four stages since in the case of the powdery product being classified such that all of the particles were less than 350 µm in particle size, it is required not to be consolidated for at least two weeks, particularly preferably for one month or more.
⊚ : No consolidation was observed for three months
○ : No consolidation was observed for one month
□ : No consolidation was observed for two weeks.
× : Consolidation occurred within less than two weeks

As shown in Table 5, in the case of the powdery product being classified to all less than 350 µm in particle size, it was found that no consolidation was observed at least for two weeks when the content of fine powder having a moisture content of 2.0% by weight or a particle size of less than 150µm was 30% or less. Furthermore, when the content of the fine powder having a moisture content of 2.0% by weight or less and having a particle size of less than 150 µm was 30% or less or when the content of the fine powder having a moisture content of 0.5% by weight or less and a particle size of less than 150 µm was 40% or less, it was found that no consolidation was observed at least for one month. Furthermore, after that, the consolidation state was successively observed. However, when the content of fine powder having a moisture content of 1.0% by weight or less and a particle size of less than 150 µm was 20% or less or when the content of fine powder having a moisture content of 0.5% by weight or less and a particle size of less than 150 µm was 30% or less, no consolidation was observed even after three months.

**[Table 5]**

| Evaluation on non-consolidation property of powdery product having particle size of less than 350 µm | | | | |
|---|---|---|---|---|
| | | Content of part having particle size of less than 150 µm | | |
| | | 20% | 30% | 40% |
| Moisture content | 0.5 weight % | ⊚ | ⊚ | ○ |
| | 1.0 weight % | ⊚ | ○ | □ |
| | 2.0 weight % | ○ | ○ | □ |
| | 3.0 weight % | □ | □ | × |

### Example 14 (Evaluation on non-consolidation property of powdery product having particle size of less than 350 µm)

Powder containing an eutectic crystalline sorbitol/maltitol each having an addition rate of seed of 25% by weight and 35% by weight were prepared by the same way as that of Example 12 and then pulverized with a hammer mill, followed by classification, respectively. Therefore, powders all having a particle size of less than 350 µm (powdery products having a particle size of less than 350 µm) were obtained. Subsequently, it was adjusted to have a moisture content of 1.0% by weight and then subjected to measurement with differential scanning calorimetry. The powder containing an eutectic crystalline sorbitol/maltitol having a seed addition rate of 25% by weight had a melting calorie of 68.5 J/G and one having a seed addition rate of 35% by weight had a melting calorie of 73.3 J/G. The DSC curve of the powder added with 35% by weight of the seed is illustrated in Fig. 10. The parts having a particle size of less than 150 µm were adjusted to 20%, 30%, and 40%, respectively. Then, 20 kg of each powder was measured and then placed and sealed in a plastic bag. Subsequently, the plastic bag filled with the powder was packaged in a corrugated board box. Each of the boxes was periodically opened to confirm the consolidation state of the powders.
The consolidation state was evaluated based on the following four stages.
⊚ : No consolidation was observed for three months
○ : No consolidation was observed for one month
□ : No consolidation was observed for two weeks
× : Consolidation occurred within less than two weeks

As shown in Table 6, in the case of the powdery product having a particle size of all less than 350µm, it was confirmed that the powder containing an eutectic crystalline sorbitol/maltitol having a melting calorie of 70 J/G or more was hardly consolidated, compared with the powder containing an eutectic crystalline sorbitol/maltitol having a melting calorie of less than 70 J/G. Furthermore, when the melting calorie was set to 70 J/G or more and the content of the part having a particle size of less than 150 µm was adjusted to 30% or less, no consolidation was observed even after three months.
As a result, with respect to the powdery product having a particle size of less than 350 µm, it was confirmed that the powder containing an eutectic crystalline sorbitol/maltitol having a melting calorie of 70% or more was hardly consolidated, compared with the powder containing an eutectic crystallinesorbitol/maltitol having a melting calorie of less than 70%.

**[Table 6]**

| Evaluation of non-consolidation property of powdery product having particle size of less than 350 µm | | | | |
|---|---|---|---|---|
| | | Content of fine powder having particle size of less than 150 µm | | |
| | | 20% | 30% | 40% |
| Melting calorie | 73.3 J/G | ⊚ | ⊚ | ○ |
| | 68.5 J/G | ⊚ | ○ | □ |

From the above results, when the moisture content was 2.0% by weight or less, the content of the fine powder having a particle size of less than 150 µm was 30% or less, or when a melting calorie was 70 J/G or more, a granulated product being classified to all less than 2 mm in particle size was hardly consolidated compared with others regardless of the case where the powdery product has a particle size of all less than 350 µm.

### Example 15 (Investigation of rate of dissolution)

Powder containing an eutectic crystalline sorbitol/maltitol prepared by the same way as that of Example 11 was classified into a part (crude particle part) having a particle size of 350 µm or more but less than 2 mm and a part (powdery product of 150 to 350. µm in particle size) having a particle size of 150 µm or more but less than 350µm.
In addition, as a control, powdery sorbitol (SORBITOL FP, manufactured by NIKKEN FINE CHEMICALS CO., LTD.), powdery maltitol (AMARTY MR, manufactured by TOWA KASEI CO., LTD.), and a mixture of powdery sorbitol andpowderymaltitol (1:1), which were classified so as to have a particle size of 150 µm or more but less than 350 µm (powdery product of 150 to 350 µm in particle size), were prepared.
Ten grams of each of them was poured into 100 ml of water at 20°C and then subjected to the measurement of dissolving time. Consequently, the crude part of the powder containing an eutectic crystalline sorbitol/maltitol dissolved within 50 seconds, the powdery product of the powder containing an eutectic crystalline sorbitol/maltitol having a particle size of 150 to 350 µm dissolved within 18 seconds, the powdery sorbitol dissolved within 35 seconds, the powdery maltitol dissolved within 54 seconds, and the mixture of the powdery sorbitol and the powdery maltitol dissolved within 46 seconds.
As a result, the powdery product of the powder containing an eutectic crystalline sorbitol/maltitol having a particle size of 150 to 350 µm showed a higher rate of dissolution, compared with that of the crude part of the powder containing an eutectic crystalline sorbitol/maltitol. When making a comparison between those having the same particle size, the powder containing an eutectic crystalline sorbitol/maltitol showed the highest rate of dissolution among the tested products.

### Example 16 (Evaluation of non-consolidation property with respect to powdery products each containing different contents of high molecular sugar alcohol)

Hydrogenated starch hydrolysate having a sugar composition of 44% by weight of sorbitol, 50% by weight of maltitol, and 6% by weight of high molecular sugar alcohol (including 4% by weight of maltotriitol and 2% by weight of a high molecular part not smaller than maltotetraitol),hydrogenatedstarch hydrolysate having a sugar composition of 43% by weight of sorbitol, 49% by weight of maltitol, and 8% by weight of high molecular sugar alcohol (including 5% by weight of maltotriitol and 3% by weight of a high molecular part not smaller than maltotetraitol), and hydrogenated starch hydrolysate having a sugar composition of 41% by weight of sorbitol, 48% by weight of maltitol, and 11% by weight of high molecular sugar alcohol (including 7% by weight of maltotriitol and 4% by weight of a high molecular part not smaller than maltotetraitol) were pulverized with a seed addition rate of 35% by weight by the same way as that of Example 13, respectively, thereby obtaining powdery products each having a particle size of less than 350 µm. Each of the powdery products was adjusted to have a moisture content of 1.0% by weight. Fine powders (less than 150 µm in particle size) of the powder containing an eutectic crystalline sorbitol/maltitol were adjusted to 20%, 30%, and 40%, respectively. Then, 20 kg of each powder was measured and then placed and sealed in a plastic bag. Subsequently, the plastic bag filled with the powder was packaged in a corrugated board box. Each of the boxes was periodically opened to confirm the consolidation state of the powders.
The consolidation state (non-consolidation property) was evaluated based on the following four stages.
⊚ : No consolidation was observed for three months
○ : No consolidation was observed for one month
□ : No consolidation was observed for two weeks
× : Consolidation occurred within less than two weeks

As shown in Table 7, in the case of the powdery product having a particle size of all less than 350 µm, it was confirmed that consolidation was hardly occurred in the powder containing an eutectic crystalline sorbitol/maltitol in which the content of high molecular sugar alcohol, which was not smaller than maltotriitol, was less than 10% by weight, compared with the powder containing an eutectic crystalline sorbitol/maltitol in which the content of high-molecular sugar alcohol, which was not smaller than maltotriitol, was 10% by weight or more.

**[Table 7]**

| Evaluation on non-consolidation property of powdery products having different contents of high molecular sugar alcohol | | | | |
|---|---|---|---|---|
| | | Content of fine powder having particle size of less than 150 µm | | |
| | | 20% | 30% | 40% |
| Content of high molecular sugar alcohol | 6% by weight | ⊚ | ⊚ | ○ |
| | 8% by weight | ⊚ | ⊚ | ○ |
| | 11% by weight | ⊚ | ○ | □ |

### Example 17 (Production of composition containing eutectic crystalline sorbitol/maltitol)

The eutectic crystalline sorbitol/maltitol obtained in Example 1 was added to 100g of a viscous liquid composition prepared such that an aqueous sugar alcohol solution containing 70% sorbitol and 30% maltitol was added with 0.24% of ACESULFAME-K, which is a commercially available high-intensity sweetener, with respect to the solid content of the aqueous sugar alcohol solution and condensed under reduced pressure to a moisture content of 1% or less, in the same manner with Example 1. Subsequently, the resulting mixture was kneaded at 80°C for about 15 minutes and then stored in a thermostatic chamber at 50°C to carry out aging. After carrying out the aging for 12 hours, the mixture was pulverized with a hammer type pulverizer, thereby obtaining a powder of a composition containing an eutectic crystalline sorbitol/maltitol without clogging the pulverizer.
The resulting powder had almost the same degree of sweetness as that of sugar and good feeling of dissolving in the mouth with a refreshing quality of sweetness.

### Industrial applicability

According to the present invention, an eutectic crystalline sugaralcoholcan be efficiently produced. In addition,theeutectic crystalline sugar alcohol thus obtained has a good feeling of dissolving in the mouth and a favorable quality of sweetness as well as excellent characteristics of uniformity, grindability, low hygroscopicity, and solubility. Therefore, it can be used widely for food products, medical products and cosmetic products as well as in industrial field.

## Claims

1. A method of manufacturing an eutectic crystalline sugar alcohol that represents a single melting peak obtained by differential scanning calorimetry, comprising:
allowing a liquid composition containing two or more kinds of the sugar alcohol at a predetermined ratio to be added with a powder containing crystals of the same two or more kinds of the sugar alcohol at substantially the same usage ratio as that of the sugar alcohol; and
kneading and aging a resulting mixture.

2. A method of manufacturing an eutectic crystalline sugar alcohol according to claim 1, wherein the sugar alcohol includes at least one or more kinds of disaccharide sugar alcohol.

3. A method of manufacturing an eutectic crystalline sorbitol/maltitol that represents a single melting peak at a temperature of 91±5°C obtained by differential scanning calorimetry, comprising:
allowing a liquid composition containing sorbitol in an amount of 51 to 80% by weight and maltitol in an amount of 49 to 20% by weight to be added with a powder containing a sorbitol crystal and a maltitol crystal at the same usage ratio as that of the sorbitol and the maltitol, respectively; and
kneading and aging a resulting mixture.

4. An eutectic crystalline sugar alcohol that represents a single melting peak obtained by differential scanning calorimetry, obtained by:
allowing a liquid composition containing two or more kinds of the sugar alcohol at a predetermined ratio to be added with a powder containing crystals of the same two or more kinds of the sugar alcohol at substantially the same usage ratio as that of the sugar alcohol; and
kneading and aging a resulting mixture.

5. An eutectic crystalline sugar alcohol according to claim 4, wherein the sugar alcohol includes at least one or more kinds of disaccharide sugar alcohol.

6. An eutectic crystalline sorbitol/maltitol that represents a single melting peak at a temperature of 91 ± 5°C obtained by differential scanning calorimetry, obtained by:
allowing a liquid composition containing sorbitol in an amount of 51 to 80% by weight and maltitol in an amount of 49 to 20% by weight to be added with a powder containing a sorbitol crystal and a maltitol crystal at the same usage ratio as that of the sorbitol and the maltitol, respectively; and
kneading and aging a resulting mixture.

7. An eutectic crystalline sugar alcohol that represents a single melting peak obtained by differential scanning calorimetry according to any one of claims 4 to 6, wherein the percentage content of the powder having a particle size of less than 350 µm is 95% or more.

8. A powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, wherein a moisture content is 2% by weight or less.

9. A powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, wherein a percentage content of a fine powder having a particle size of less than 150 µm is 30% or less.

10. A powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, wherein a melting calorie is 70J/g or more.

11. A powder containing an eutectic crystalline sorbitol/maltitol, comprising a powdery composition that contains an eutectic crystalline sorbitol/maltitol including sorbitol in an amount of 40 to 80% by weight and maltitol in an amount of 60 to 20% by weight and has a melting peak at a temperature of 91 ± 5°C obtained by differential scanning calorimetry, wherein a moisture content is 2% by weight or less, and/or a percentage content of a fine powder having a particle size of less than 150 µm is 30% or less, and/or a melting calorie is 70J/g or more.

12. A powder containing an eutectic crystalline sorbitol/maltitol according to any one of claims 8 to 11, wherein the percentage content of a powder having a particle size of less than 350 µm is 95% or more.

13. A powder containing an eutectic crystalline sorbitol/maltitol according to any one of claims 8 to 12, wherein the content of the sorbitol is 40 to 60% by weight and the content of the maltitol is 60 to 40% by weight, and a content of high molecular sugar alcohol, which is not smaller than maltotriitol, is less than 10% by weight.

14. A method of manufacturing a composition containing an eutectic crystalline sugar alcohol, comprising an eutectic crystalline sugar alcohol that represents a single melting peak obtained by differential scanning calorimetry, and ingredients of food products, food additives, pharmaceutical excipients pharmaceutical products, or cosmetic products, wherein:
a liquid composition containing two or more kinds of the sugar alcohol at a predetermined ratio is added with ingredients of food products, food additives, pharmaceutical excipients, pharmaceutical products, or cosmetic products, followed by addition of a powder containing crystals of the same two or more kinds of the sugar alcohol at substantially the same usage ratio as that of the sugar alcohol, and kneading and aging a resulting mixture; or
a liquid composition containing two or more kinds of the sugar alcohol at a predetermined ratio is added with a powder containing crystals of the same two or more kinds of the sugar alcohol at substantially the same usage ratio as that of the sugar alcohol, simultaneously with addition of ingredients of food products, food additives, pharmaceutical excipients, pharmaceutical products, or cosmetic products, followed by kneading and aging.

15. A method of manufacturing a powdery composition, comprising a powder containing an eutectic crystalline sorbitol/maltitol, and ingredients of food products food additives pharmaceutical excipients, pharmaceutical products, or cosmetic products, wherein:
a liquid composition in which the content of the sorbitol is 40 to 80% by weight and the content of the maltitol is 60 to 20% by weight is added with ingredients of food products, food additives, pharmaceutical excipients, pharmaceutical products, or cosmetic products, followed by addition of a powder containing a sorbitol crystal and a maltitol crystal at the same usage ratio as that of the sorbitol and the maltitol, respectively, and kneading and aging a resulting mixture; or
a liquid composition in which the content of the sorbitol is 40 to 80% by weight and the content of the maltitol is 60 to 20% by weight is added with a powder containing a sorbitol crystal and a maltitol crystal substantially at the same usage ratio as that of the sorbitol and maltitol, respectively, simultaneously with addition of ingredients of food products, food additives, pharmaceutical excipients, pharmaceutical products, or cosmetic products, followed by kneading and aging.

16. A composition containing an eutectic crystalline sugar alcohol which is produced by a method according to claim 14, comprising an eutectic crystalline sugar alcohol that represents a single melting peak obtained by differential scanning calorimetry, and ingredients of food products, food additives, pharmaceutical excipients, pharmaceutical products, or cosmetic products.

17. A powdery composition which is produced by a method according to claim 15, comprising a powder containing an eutectic crystalline sorbitol/maltitol, and ingredients of food products, food additives, pharmaceutical excipients, pharmaceutical products, or cosmetic products.
